# EUROPEAN PATENT SPECIFICATION

(11) **EP 2 902 293 B1**
(45) Date of publication and mention of the grant of the patent: **04.03.2020**
(21) Application number: 15157469.6
(22) Date of filing: 12.11.2009
(51) Int. Cl.: B60W 40/00, B60W 40/06, B60W 40/12, B60C 23/00, B60C 23/04

(54) **METHODS OF MAKING EXTREMELY STRETCHABLE ELECTRONICS**
HERSTELLUNG EXTREM DEHNBARER ELEKTRONIK
PROCÉDÉ DE FABRICATION DES DISPOSITIFS ÉLECTRONIQUES EXTRÊMEMENT ÉTIRABLES

(30) Priority: 12.11.2008 US 113622 P; 07.10.2009 US 575008
(43) Date of publication of application: 05.08.2015
(62) Divisional of application: 09826745.3
(73) Proprietor: Mc10, Inc., Waltham, MA 02451 (US)
(72) Inventor: Arora, William, Bellevue, WA 98004 (US); Ghaffari, Roozbeth, Cambridge, MA 024142 (US)
(74) Representative: Mewburn Ellis LLP

(56) References cited:
- WO-A1-99/38211
- WO-A1-2007/116344
- WO-A2-03/021679
- US-A1- 2002 094 701
- US-A1- 2004 192 082
- US-A1- 2006 071 349
- US-B1- 6 775 906

## Description

### CROSS-REFERENCE TO RELATED APPLICATIONS

This application claims the benefit of United States Provisional Application No. 61/113,622 entitled "Extremely Stretchable Interconnects" filed on November 12, 2008. Also, this application is a continuation-in-part of, and claims the benefit of United States Non-Provisional Application No. 12/575,008, entitled "Catheter Balloon Having Stretchable Integrated Circuitry and Sensor Array" filed on October 7, 2009. Application No. 12/575,008 claimed the priority of United States Provisional Application Nos. 61/103,361, filed October 7, 2008 and 61/113,007, filed November 10, 2008.

### FIELD OF THE INVENTION

The present invention relates to methods of assembling expandable or stretchable integrated circuitry, more particularly extremely stretchable integrated circuitry.

### BACKGROUND OF THE INVENTION

The field of stretchable electronics continues to grow due to the demand of high performance and mechanically unconstrained applications of the future. However, stretchable electronics have been thus far limited in stretchability. This has limited the ability of stretchable electronics to accommodate applications that require more extreme stretchability. Therefore a need exists for extremely stretchable electronics.

United States Patent Application Publication Number US 2002/0094701 A1 shows sensor arrays employing stretchable interconnects, made of coiled conductors, for electrically connecting electronic devices. The interconnects comprising photolithographically patterned conductors extending between two devices, electrically coupling them together. The coiled conductor is formed of a metal or alloy having a stress gradient extending through a thickness of the conductor.

International Patent Application Publication Number WO 2007/116344 A1 shows an integrated circuit device having several rigid substrate islands containing circuit components connected by elastically deformable conductive connections each containing a signalling layer.

International Patent Application Publication Number WO 99/38211 A1 shows a flexible film having semiconductor elements attached, those semiconductor elements being electrically connected by a connection line. The semiconductor substrate on which the elements are produced is etched away leaving the flexible film as the only mechanical connection between the semiconductor elements.

United States Patent Application Publication Number US 2006/071349 A1 shows a semiconductor device having a flexible, curved substrate, an external connection electrode and a semiconductor element, the element being electrically connected to the electrode by a wire.

United States Patent Application Publication Number US 2004/192082 A1 shows stretchable interconnects of various geometric configurations. The interconnects are formed of an electrically conducting film or an elastomer material to provide elastic properties.

International Patent Application Publication Number WO 03/021679 A2 shows a mechanically flexible integrated circuit structure, which can be incorporated into a fabric, comprising a plurality of microelectronic elements that are separate fabrications interconnected by a flexible interconnect system. That system including electrical interconnections of copper wire and a mechanical interconnection element of a flexible polymer.

United States Patent Number US6775906 is a method of manufacturing an integrated circuit carrier, wherein a receiving zone for an integrated circuit is demarcated on a substrate, island-defining portions are arranged about each of the receiving zones and rigidity reducing arrangements are created between neighbouring island-defining portions by removing material from the substrate.

### SUMMARY OF THE INVENTION

This invention concerns methods of making extremely stretchable interconnects. In embodiments, the invention comprises a method of making stretchable electronics, which in some embodiments can be out of high quality single crystal semiconductor materials or other semiconductor materials, that are typically rigid. For example, single crystal semiconductor materials are brittle and cannot typically withstand strains of greater than about +/- 2%. This invention describes a method of electronics that are capable of stretching and compressing while withstanding high translational strains, such as in the range of -100,000% to +100,000%, and/or high rotational strains, such as to an extent greater than 180°, while maintaining electrical performance found in their unstrained state.

In embodiments, the stretching and compressing may be accomplished by fabricating integrated circuits (ICs) out of thin membrane single crystal semiconductors, which are formed into "islands" that are mechanically and electrically connected by "interconnects," and transferring said ICs onto an elastomeric substrate capable of stretching and compressing. The islands are regions of non-stretchable/compressible ICs, while the interconnects are regions of material formed in a way to be highly stretchable/compressible. The underlying elastomeric substrate is much more compliant than the islands, so that minimal strain is transferred into the islands while the majority of the strain is transferred to the interconnects, which only contain electrical connections and not ICs. Each interconnect attaches one island to another island, and is capable of accommodating strain between the two aforementioned islands, including translation, rotation, or a combination of translation with rotation of one island relative to another. Even though the interconnects may be made of a rigid material, they act like weak springs rather than rigid plates or beams. This configuration thereby allows for the making of extremely stretchable electronics.

These and other systems, methods, objects, features, and advantages of the present invention will be apparent to those skilled in the art from the following detailed description of the preferred embodiment and the drawings.

### BRIEF DESCRIPTION OF THE DRAWINGS

The invention and the following detailed description of certain embodiments thereof may be understood by reference to the following figures:
Figure 1 depicts an overhead view of an embodiment of the present invention showing two device islands connected edge-to-edge by a monolithically formed extremely stretchable interconnect, prior to being stretched.
Figure 2 depicts an overhead view of an embodiment of the present invention showing two device islands connected edge-to-edge by two extremely stretchable interconnects.
Figure 3 depicts an overhead view of an embodiment of the present invention showing two device islands connected edge-to-edge by three extremely stretchable interconnects; in this case, the long bars of the interconnects are rotated by 90° which allows them to be longer than if they were not rotated.
Figure 4 depicts four device islands arranged in a square matrix in an embodiment of the present invention, with each edge connected by an extremely stretchable interconnect to its nearest neighbors island edge, and the interconnects are formed so as to maximize the amount of chip area that is used for either an island or interconnect.
Figure 5 depicts the case of Figure 1, with the short bars widened for extra mechanical strength at those locations.

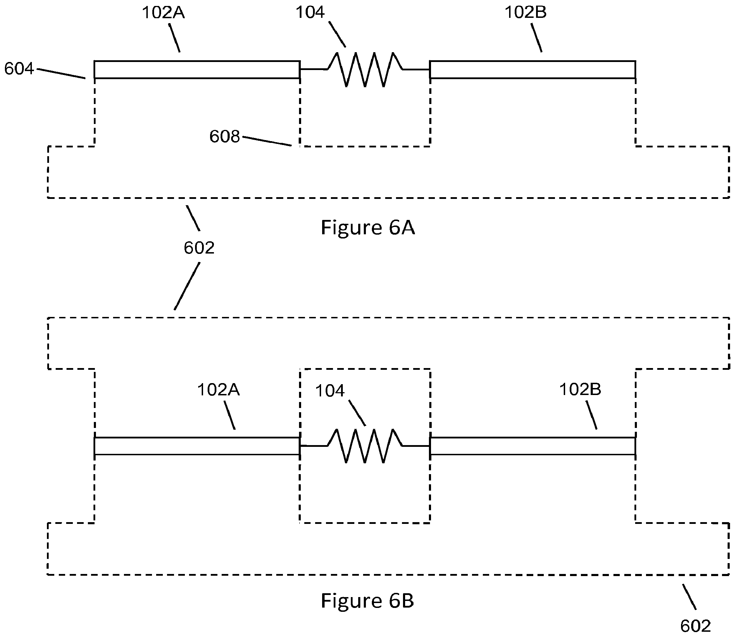

Figure 6 depicts embodiments of the present invention, where (a) is a side view of device islands and extremely stretchable interconnects transferred onto an elastomeric substrate. In this case, the substrate has been molded to have posts that are of the same area as the device islands (note that in embodiments these could be smaller or larger than the device islands). The height "h" of the molded post regions may range from, but is not limited to, about 1-1000 µm. The interconnects are located in between these regions as shown. (b) Side view as before, with a similarly shaped elastomeric superstrate to serve as an encapsulation layer protecting the devices from direct mechanical contact.
Figure 7 depicts a side view of a two-layer PDMS substrate in an embodiment of the present invention comprising silicon device islands adhered to top layer, free-standing interconnects, and square wave ripples in the lower layer PDMS to promote increased stretching through the substrate.
Figure 8 depicts an embodiment of the present invention with a side view of two layers of cured photoresist (SU-8 50 and SU-8 2002) used to make the two-layer PDMS substrate described in Figure 7.
Figure 9 depicts an embodiment of the present invention with a side view of a two-layer PDMS substrate consisting of sinusoidal waves in the lower layer of PDMS to promote increased stretching through the substrate.

### DETAILED DESCRIPTION OF THE INVENTION

The present invention accomplishes extremely stretchable electronics by forming the electronics on discrete islands 102 of silicon.

With reference to the present invention, the term "stretchable", and roots and derivations thereof, when used to modify circuitry or components thereof is meant to encompass circuitry that comprises components having soft or elastic properties capable of being made longer or wider without tearing or breaking, and it is also meant to encompass circuitry having components (whether or not the components themselves are individually stretchable as stated above) that are configured in such a way so as to accommodate and remain functional when applied to a stretchable, inflatable, or otherwise expandable surface. The term "expandable", and roots and derivations thereof, when used to modify circuitry or components thereof is also meant to have the meaning ascribed above. Thus, "stretch" and "expand", and all derivations thereof, may be used interchangeably when referring to the present invention.

In embodiments, the discrete islands mention above are discrete operative (in embodiments, arranged in a "device island" arrangement) and are themselves capable of performing the functionality described herein, or portions thereof. In embodiments, such functionality of the operative devices can include integrated circuits, physical sensors (e.g. temperature, pH, light, radiation etc), biological and/or chemical sensors, amplifiers, A/D and D/A converters, optical collectors, electro-mechanical transducers, piezo-electric actuators, light emitting electronics which include LEDs, and combinations thereof. The purpose and advantage of using standard ICs (in embodiments, CMOS, on single crystal silicon) is to have and use high quality, high performance, and high functioning circuit components that are also already commonly mass-produced with well known processes, and which provide a range of functionality and generation of data far superior to that produced by a passive means.

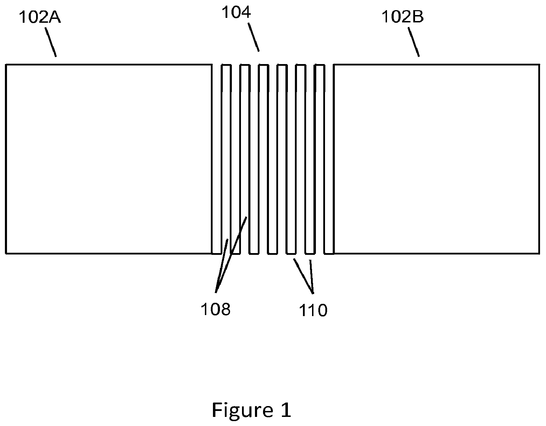

In an example, the discrete islands 102 may range from about, but not limited to, 10-100 µm in size measured on an edge or by diameter, and connecting said islands 102A-B with one or more extremely stretchable interconnects 104. The novel geometry of the interconnects 104 is what makes them extremely compliant. Each interconnect 104 is patterned and etched so that its structural form has width and thickness dimensions that may be of comparable size (such as their ratio or inverse ratio not exceeding about a factor of 10); and may be preferably equal in size. In embodiments, the dimensions may not be greater than about 5 µm (e.g. where both dimensions are about 1 µm or less). The interconnect 104 may be formed in a boustrophedonic style such that it effectively comprises long bars 108 and short bars 110 as shown in Figure 1. This unique geometry minimizes the stresses that are produced in the interconnect 104 when subsequently stretched because it has the effective form of a wire, and behaves very differently than interconnect form factors having one dimension greatly exceeding the other two (for example plates). Plate type structures primarily relieve stress only about a single axis via buckling, and withstand only a slight amount of shear stress before cracking. This invention may relieve stress about all three axes, including shears and any other stress.

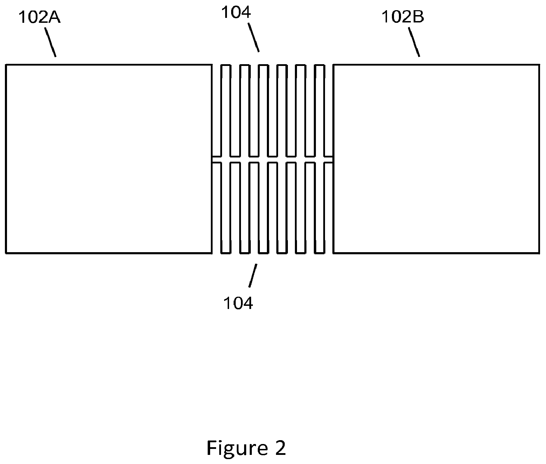

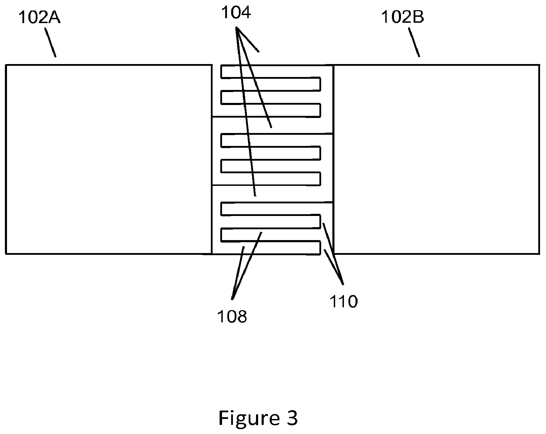

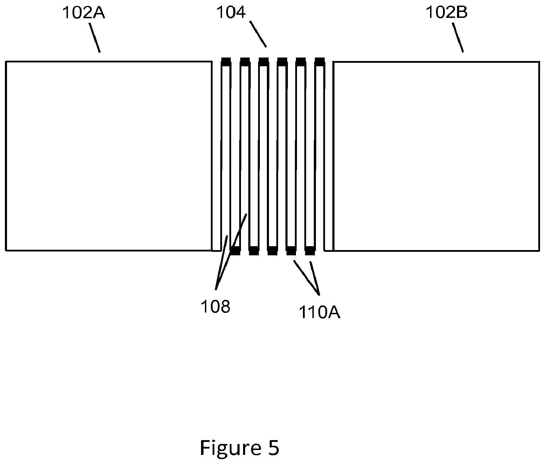

In addition, because the interconnect 104 may be formed out of rigid materials, after being stretched it may have a restorative force which helps prevent its wire-like form from getting tangled or knotted when re-compressing to the unstretched state. Another advantage of the boustrophedonic geometry is that it minimizes the initial separation distance between the islands 102A-B. This is illustrated in Figure 1. One or more interconnects 104 may be formed in various ways, as shown in Figures 2-4. The parts of the interconnect 104 where the majority of stresses build up during stretching may be the short linking bars. To minimize cracking here, the short linking bars 110A may be made several micrometers wider than the longer bars 108, as shown in Figure 5.

In embodiments, the connection point of the interconnect 104 to the device island 102 may be anywhere along the device island edge, or may be at a point on the surface of the device island 102 (in which case the interconnect may be located just above the plane of the device island).

In embodiments, device islands 102 may be made on any suitable material substrate, provided that a top membrane layer of said substrate that contains the ICs can be freed from the bulk of the substrate and transfer printed onto an elastomeric substrate.

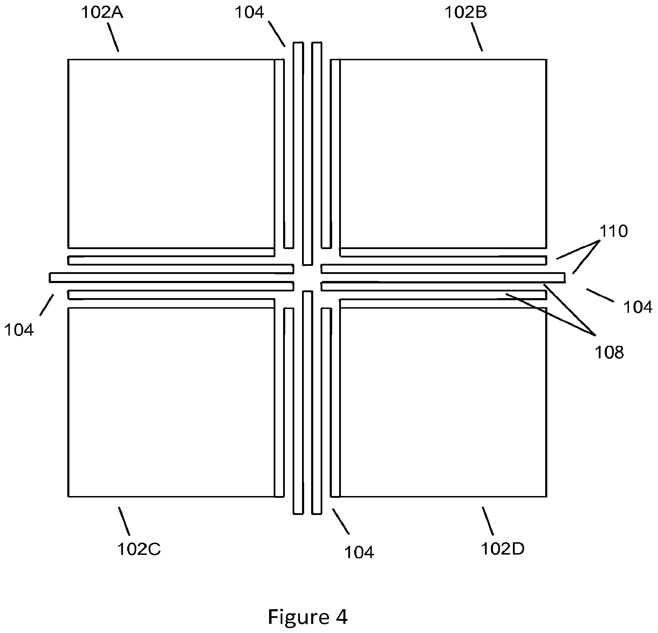

In the present invention, the interconnects 104 (as described herein) may be formed either monolithically (i.e., out of the same semiconductor material as the device islands) or may be formed out of another material. In one non-limiting example embodiment, the stretchable electronics are fabricated on a silicon-on-insulator (SOI) wafer, having a 1 µm thick top silicon layer and a 1µm thick buried oxide layer. Devices are formed on the top silicon wafer, and arranged into a square pattern of islands 102A-D and interconnects 104 of the general form shown in Figure 4, in which the islands 102 are 100 µm on an edge, and the interconnects 104 are 1 µm wide, and the space between each long bar is 1 µm, and the interconnects 104 comprise 10 long bars 108, all about 100 µm long. The islands 102 and interconnects 104 are formed in an etching step which removes the excess silicon. The islands 102 and interconnects 104 are coated with a 1 um layer of polyimide that is patterned to only cover the islands 102 and interconnects 104. Next, the islands 102 and interconnects 104 are released in an HF etch which undercuts the underlying buried oxide. After drying, the islands 102 and interconnects 104 are transfer printed with a Polydimethylsiloxane (PDMS) stamp onto an elastomeric substrate 602. After being picked up by the transfer stamp, and prior to being placed onto the elastomeric substrate 602, the backsides of the islands 102 may be coated with a layer of polyimide (patterned to only cover the islands 102 and interconnects 104), and an additional layer of evaporated 3 nm chromium and 30 nm silicon dioxide selectively over the island regions to improve adhesion to the elastomeric substrate 602 at those locations, and not along the interconnects 102. The elastomeric substrate 602 may be PDMS or another highly compliant material. The elastomeric substrate 602 may additionally be molded or etched into the shape shown in Figure 6A-B, to further increase selective adhesion in the device island region but not the interconnect region, and to reduce the amount of material strain in the elastomeric substrate 602 that is transferred to the device islands 102. In this example, the interconnects may accommodate stretching the device islands apart by approximately up to 800 µm. In addition, the interconnects 104 of this example may be capable of accommodating lateral shear displacements of about 800 µm. In general, they may be capable of accommodating any relative displacement of the two islands such that they remain approximately within 800 µm of each other. In addition, the interconnects 104 may accommodate corkscrew type rotations of one island relative to another about any of the three axes of rotation. This feature may be limited only by the interconnects becoming entangled within each other. In any practical application, the completed stretchable device may not be so severely rotated, and the interconnect may easily accommodate rotations of up to 180°. It is noted that by increasing the number of long bars 108 used in the interconnect 104, or by increasing the length of the long bars 108, the interconnect may be able to accommodate even larger displacement strains. In embodiments, there may be no practical upper limit to the amount of displacement enabled through the present invention.

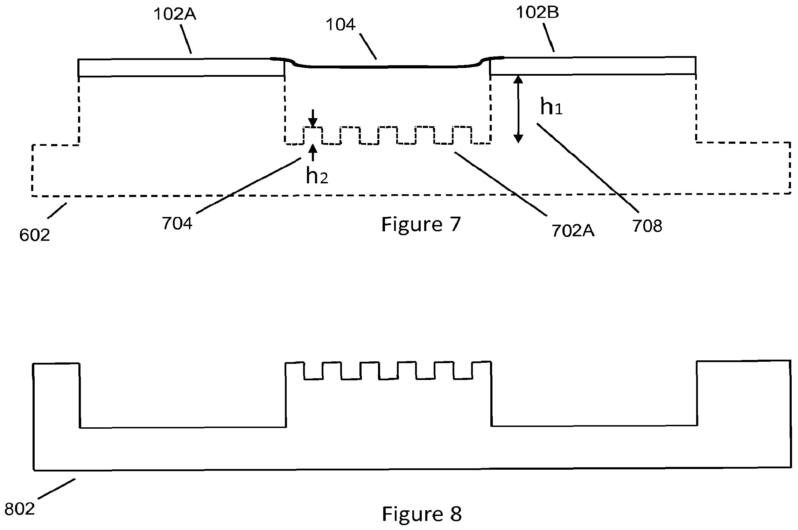

In another embodiment the elastomeric substrate 602 may comprise two layers separated by a height. The top "contact" layer contacts the device island 102 as in the embodiment illustrated in Figure 6. In addition, figure 7 shows the bottom layer 702 may be a "wavy" layer containing ripples or square waves molded into the substrate 602 during elastomer fabrication. These square waves enable additional stretching, whose extent depends on the amplitude and wavelength of the waves pattern-molded in the elastomer 602. Figure 7 shows one non-limiting layout and topology of an elastomeric substrate 602 relative to the position of the interconnects 104 and device islands 102A-B. In an example, a two layer molded substrate can be fabricated using two step process consisting of two types of negative photoresist (SU-8 50 and SU-8 2002; Microchem Corporation). The negative resists can be spin-coated on a transfer silicon wafer with spin speeds of 3000 rpm. The SU-8 50 layer can be spun on the wafer, and subsequently cured with UV radiation. Once the SU-8 50 layer has hardened, the SU-8 2002 can be spun and cured with a photo-mask and an alignment tool. In this example, the thickness of the SU-8 50 and SU-8 2002 are 40-50 µm 708 and 2-10 µm 704, respectively. The 40-50 µm thick regions of SU-8 50 contain ripples 702 of SU-8 2002 (in this instance in the form of square waves) on their surfaces. Upon curing of the SU-8 2002 layer, liquid PDMS can be poured over the SU-8 patterns to form a substrate in the shape of the SU-8 molds 802, as shown in Figure 8. The amplitude of the ripples in the SU-8 mold 802 can be varied by changing the spin speed used for spinning the thin layer of SU-8 2002. In this configuration, the interconnects 104 are free-standing. The entire substrate-device configuration can be immersed in non-cured elastomer (fluid layer) layer followed by a cured layer of PDMS to encapsulate the fluid and devices.

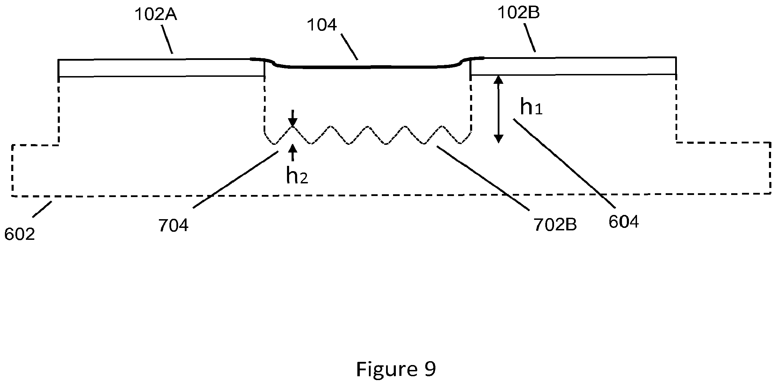

In another embodiment, the PDMS in the lower layer may be designed with periodic sinusoidal ripples 702B. In embodiments, this ripple configuration may be achieved by bonding Si nanoribbons on the surface of pre-strained PDMS in a uniform parallel pattern. The release of the prestrain in the PDMS substrate generates sinusoidal waves along the thin Si-nanoribbons (caused by buckling) and the surface of the PDMS substrate. The amplitude and wavelength of these waves 702B may depend on the extent of uniaxial pre-strain exerted on the PDMS and on the mechanical properties of the Si-nanoribbons. The wavy surface on the PDMS may be used as a transfer mold. Two-part liquid plastic solution can be poured over the wavy PDMS substrate and cured at room temperature over time (∼2 hrs). Once the plastic hardens, the plastic substrate can be peeled away from the PDMS. This new plastic transfer substrate with wavy surface features can be used to produce more PDMS substrates containing wave features. The wavy PDMS may serve as the lower layer of PDMS as in the previous embodiment. To produce a two layer PDMS structure, a top layer of PDMS can be plasma bonded to this lower layer of PDMS using oxygen plasma surface activation to produce the substrate illustrated in Figure 9.

In another embodiment, the PDMS transfer stamp is stretched after the islands 102A-B and interconnects 104 are picked up. A subsequent transfer to another elastomeric substrate 602 may place these pre-stretched devices in a configuration, which allows the new elastomeric substrate to undergo compression. The devices may be able to accommodate that compression because the interconnects are pre-stretched.

In another embodiment, the interconnects 104 are not made out of the same material as the device islands 102. In this case, the islands 102A-B are completely isolated from each other by etching, with no interconnects in between. In an example, a layer of polyimide may then be deposited, contact vias etched to various locations on the surface of the device island 102, and then metal interconnects 104 deposited and patterned into a boustrophedonic pattern, followed by another layer of polyimide. Both layers of polyimide may now be patterned and etched to leave a small border around the interconnects 104 (thereby fully encapsulating the interconnects). These interconnects may have the advantage that they are already fully encapsulated in polyimide and will not adhere as well to the elastomeric substrate as the device islands will. The other advantage is that these interconnects may not be limited to only connecting along the edge of an island. The contact via may be etched anywhere on the surface of the island 102, including near the center. This may allow for easier connections to devices, more connections than possible only along an edge, increased strain compliance, decreased strain at the contact vias, and multiple layers of interconnects made with polymer passivation layers in between, allowing even more interconnects, or allowing one device island 102A to connect to a non-neighboring device island 102B.

In another embodiment of the invention, the device islands 102 are fabricated and transfer printed onto the elastomeric substrate 602, or substrate comprising a polymeric release layer and polymeric non-release layer. After transfer printing, the interconnects 104 are formed as described above, which may be possible because they do not require any high temperature processing, and then in the latter case, the release layer is etched and the devices that are on the non-release layer, are transfer printed onto another elastomeric substrate 602. In the former case, the islands 102 may be transferred onto the elastomeric substrate using pick and place technology so that islands 102 that are initially fabricated very close to each other are spread apart when they are transfer printed. This allows the interconnects 104 to be fabricated in a pattern that resembles their stretched configuration (if desired), to allow compression.

In embodiments, the present invention may comprise a stretchable electrical interconnect 104, including an electrical interconnect 104 for connecting two electrical contacts 102A-B (e.g. device islands 102A-B), where the electrical interconnect 104 may be arranged boustrophedonicially to define rungs 108 (i.e. long bars 108) between the contacts 102A-B, and where the rungs 108 may be substantially parallel with one another and where a plurality of rungs 108 may have substantially the same length and displacement therebetween. In addition, the ratio of the length of the plurality of rungs 108 and the displacement between the plurality of rungs 108 may be large, such as at least 10:1, 100:1, 1000:1, and the like. The electrical integrity of the electrical interconnect 104 may be maintained as stretched, such as to displacements that are increased to 1000%, 10000%, 100000%, and the like during stretching. In embodiments, the rungs 108 may be substantially perpendicular to the contacts 102A-B, the interconnection 104 may have a trace width and/or inter-rung spacing ranging between 0.1-10 microns. In embodiments, the two electrical contacts 102A-B may be located on an elastomeric substrate 602, the electrical contacts 102A-B may be bonded to the substrate 602 and the interconnection 104 not bonded to the substrate 602, the electrical contacts 102A-B may be semiconductor circuits, metal contacts, and the like.

In embodiments, the present invention may comprise a stretchable electrical interconnect 104, including an electrical interconnect 104 for connecting two electrical contacts 102A-B, where the electrical interconnect 104 is arranged boustrophedonicially to define rungs 108 between the contacts 102A-B, and where the interconnect 104 maintains electrical conductivity and electrical integrity when a displacement between the contacts 102A-B is increased, such as by 1000%, 10000%, 100000%, and the like.

In embodiments, the present invention may electrically interconnect two electrical contacts 102A-B with a stretchable interconnection 104 that has the ability to twist between the two electrical contacts 102A-B by up to approximately 180 degrees while maintaining electrical integrity of the stretchable interconnection 104.

In embodiments, the present invention may be a device including a body having a stretchable surface (e.g. an elastomeric substrate 602), and a stretchable electronic circuit including (i) a first discrete operative device 102A, (ii) a second discrete operative device 102B, and (iii) a stretchable interconnect 104 connecting the first discrete operative device 102A to the second discrete operative device 102B, where the interconnect 104 may have a substantially boustrophedonic pattern and be able to maintain electrical conductivity when stretched, such as up to 1000% , 10000%, 100000%, and the like. The stretchable electronic circuit may be affixed to the stretchable surface of the body. In embodiments, the connection may be to a metal contact, to a semiconductor device, and the like. The first discrete operative device 102A, the second discrete operative device 102B, and the stretchable interconnect 104 may all be made from the same material, and that material may be a semiconductor material.

In embodiments, the present invention may attach at least two isolated electronic components (which in embodiments may be discrete operative devices) 102A-B to an elastomeric substrate 602, and arrange an electrical interconnection 104 between the components 102A-B in a boustrophedonic pattern interconnecting the two isolated electronic components 102A-B with the electrical interconnection 104. The elastomeric substrate 602 may then be stretched such that components 102A-B separate relative to one another, where the electrical interconnection 104 maintains substantially identical electrical performance characteristics that the electrical interconnection 104 had in a pre-stretched form. In embodiments, the stretching may be a translational stretching, where the separation between the isolated electronic components 102A-B increases by a percent as a result of the stretching, such as 10%, 100%, 1000%, 10000%, 100000%, and the like. The stretching may be a rotational stretching, where the rotation may be greater than a certain rotation angle, such as 90°, 180°, 270°, 360°, and the like, where the stretching may be in all three axes. In embodiments, the electrical interconnection 104 may be made from semiconducitve material. The electrical interconnection 104 may be made from the same semiconductor material as the isolated electronic components 102A-B, fabricated at the same time as the isolated electronic components 102A-B, and the like. The semiconductor material may be a single crystal semiconductor material. The electrical interconnection 104 may made of a different material than the isolated electronic components 102A-B, such as a metal. In embodiments, the interconnect material 104 may be loosely bound to the elastomeric substrate 602, not connected at all, raised above the surface of the elastomeric substrate 602, and the like. In embodiments, the at least two isolated semiconductor circuits may be fabricated on an upper surface 604 of the elastomeric substrate 602 separated by a lower surface 608 of the elastomeric substrate 602, and the electrical interconnection 104 may be fabricated at the level of the upper surface 604 of the elastomeric substrate 602. In this way, the electrical interconnection 104 may have no direct contact with the lower level 608, and thereby be substantially free from adhesion to the lower level 608 during stretching. In addition, the lower surface 608 of the elastomeric substrate 602 may include a wavy form 702, where the wavy form 704 may allow the elastomeric substrate 602 to expand during stretching.

## Claims

1. A method of assembling a stretchable electronic device, the method comprising:
attaching at least two electronic components (102A-B) to an elastomeric substrate (602);
arranging an electrical interconnection (104) made from a semiconductor material between said_at least two electronic components (102A-B) in a boustrophedonic pattern_that defines rungs (108) between said at least two electronic components (102A-B), a plurality of said rungs (108) being substantially parallel with one another and having substantially the same length and displacement therebetween, wherein a ratio of said length of said plurality of said rungs (108) and said displacement between said plurality of said rungs (108) is at least 10:1;
interconnecting the at least two electronic components (102A-B) with said electrical interconnection (104); and
stretching the elastomeric substrate (602) such that said at least two electronic components (102A-B) separate relative to one another,.

2. The method of claim 1, wherein the stretching of the elastomeric substrate (602) is a translational stretching.

3. The method of claim 1 or 2, wherein the separation between the at least two electronic components (102A-B) increases by a percent as a result of the stretching of the elastomeric substrate (602).

4. The method of any preceding claim , wherein the semiconductor material and the boustrophedonic pattern of said electrical interconnection (104) allows said electrical interconnection (104) to maintain substantially identical electrical performance characteristics when the separation between the at least two electronic components (102A-B) increases by 10%.

5. The method of claim 1, 2 or 3, wherein the semiconductor material and the boustrophedonic pattern of said electrical interconnection (104) allows said electrical interconnection (104) to maintain substantially identical electrical performance characteristics when the separation between the at least two electronic components (102A-B) increases by100%.

6. The method of claim 1, 2, or 3, wherein the semiconductor material and the boustrophedonic pattern of said electrical interconnection (104) allows said electrical interconnection (104) to maintain substantially identical electrical performance characteristics when the separation between the at least two electronic components (102A-B) increases by1,000%.

7. The method of claim 1, 2 or 3, wherein the semiconductor material and the boustrophedonic pattern of said electrical interconnection (104) allows said electrical interconnection (104) to maintain substantially identical electrical performance characteristics when the separation between the at least two electronic components (102A-B) increases by10,000%.

8. The method of any preceding claim, wherein the rungs (108) comprise long bars (108) connected by short bars (110).

9. The method of any preceding claim, wherein the stretching of the elastomeric substrate (602) is a rotational stretching.

10. The method of claim 9, wherein the rotational stretching is greater than 180 degrees.

11. The method of any preceding claim, wherein the stretching of the elastomeric substrate (602) comprises stretching the elastomeric substrate (602) in all three axes.

12. The method of any preceding claim, wherein the at least two electronic components (102A-B) are made from semiconductive material.

13. The method of claim 12, wherein the semiconductor material of the electrical interconnection (104) is the same as the semiconductor material from which the at least two electronic components (102A-B) are made.

14. A method of assembling a stretchable electronic device, the method comprising:
attaching at least two electronic components (102A-B) to an elastomeric substrate (602);
arranging an electrical interconnection (104) made from a semiconductor material between the at least two electronic components (102A-B) in a boustrophedonic pattern which defines interconnected rungs (108) between the at least two electronic components (102A-B), wherein the electrical interconnection (104) is fabricated at the same time as the electronic components (102A-B)
interconnecting the at least two electronic components (102A-B) with the electrical interconnection (104); and
stretching the elastomeric substrate (602) such that the at least two electronic components (102A-B) separate relative to one another.

15. The method of claim 13, wherein the semiconductor material is a single crystal semiconductor material.

## Patentansprüche

1. Verfahren zum Zusammenbau einer dehnbaren elektronischen Vorrichtung, wobei das Verfahren Folgendes umfasst:
Befestigen von zumindest zwei elektronischen Komponenten (102A-B) an einem elastomeren Substrat (602);
Anordnen einer elektrischen Zwischenverbindung (104), die aus einem Halbleitermaterial besteht, zwischen den zumindest zwei elektronischen Komponenten (102A-B) in einem bustrophedonen Muster, das Querstäbe (108) zwischen den zumindest zwei elektronischen Komponenten (102A-B) definiert, wobei eine Vielzahl der Querstäbe (108) im Wesentlichen parallel zueinander steht und im Wesentlichen die gleiche Länge und die gleiche Verlagerung zwischen ihnen aufweist, wobei ein Verhältnis der Länge der Vielzahl der Querstäbe (108) und der Verlagerung zwischen der Vielzahl von Querstäbe (108) zumindest 10:1 beträgt;
Zwischenverbinden der zumindest zwei elektronischen Komponenten (102A-B) mittels der elektrischen Zwischenverbindung (104); und
Dehnen des elastomeren Substrats (602), sodass sich die zumindest zwei elektronischen Komponenten (102A-B) relativ voneinander entfernen.

2. Verfahren nach Anspruch 1, wobei das Dehnen des elastomeren Substrats (602) ein translatorisches Dehnen ist.

3. Verfahren nach Anspruch 1 oder 2, wobei das Entfernen der zumindest zwei elektronischen Komponenten (102A-B) voneinander als Folge der Dehnung des elastomeren Substrats (602) prozentuell zunimmt.

4. Verfahren nach einem der vorangegangenen Ansprüche, wobei das Halbleitermaterial und das bustrophedone Muster der elektrischen Zwischenverbindung (104) ermöglichen, dass die elektrische Zwischenverbindung (104) im Wesentlichen identische elektrische Leistungseigenschaften beibehält, wenn das Entfernen der zumindest zwei elektronischen Komponenten (102A-B) voneinander um 10 % zunimmt.

5. Verfahren nach Anspruch 1, 2 oder 3, wobei das Halbleitermaterial und das bustrophedone Muster der elektrischen Zwischenverbindung (104) ermöglichen, dass die elektrische Zwischenverbindung (104) im Wesentlichen identische elektrische Leistungseigenschaften beibehält, wenn das Entfernen der zumindest zwei elektronischen Komponenten (102A-B) voneinander um 100 % zunimmt.

6. Verfahren nach Anspruch 1, 2 oder 3, wobei das Halbleitermaterial und das bustrophedone Muster der elektrischen Zwischenverbindung (104) ermöglichen, dass die elektrische Zwischenverbindung (104) im Wesentlichen identische elektrische Leistungseigenschaften beibehält, wenn das Entfernen der zumindest zwei elektronischen Komponenten (102A-B) voneinander um 1.000 % zunimmt.

7. Verfahren nach Anspruch 1, 2 oder 3, wobei das Halbleitermaterial und das bustrophedone Muster der elektrischen Zwischenverbindung (104) ermöglichen, dass die elektrische Zwischenverbindung (104) im Wesentlichen identische elektrische Leistungseigenschaften beibehält, wenn das Entfernen der zumindest zwei elektronischen Komponenten (102A-B) voneinander um 10.000 % zunimmt.

8. Verfahren nach einem der vorangegangenen Ansprüche, wobei die Querstäbe (108) lange Stäbe (108) umfassen, die durch kurze Stäbe (110) verbunden sind.

9. Verfahren nach einem der vorangegangenen Ansprüche, wobei das Dehnen des elastomeren Substrats (602) ein drehendes Dehnen ist.

10. Verfahren nach Anspruch 9, wobei das drehende Dehnen ein Dehnen um mehr als 180 Grad ist.

11. Verfahren nach einem der vorangegangenen Ansprüche, wobei das Dehnen des elastomeren Substrats (602) das Dehnen des elastomeren Substrats (602) in allen drei Achsen umfasst.

12. Verfahren nach einem der vorangegangenen Ansprüche, wobei die zumindest zwei elektronischen Komponenten (102A-B) aus Halbleitermaterial bestehen.

13. Verfahren nach Anspruch 12, wobei das Halbleitermaterial der elektrischen Zwischenverbindung (104) dasselbe ist wie das Halbleitermaterial, aus dem die zumindest zwei elektronischen Komponenten (102A-B) bestehen.

14. Verfahren zum Zusammenbau einer dehnbaren elektronischen Vorrichtung, wobei das Verfahren Folgendes umfasst:
Befestigen von zumindest zwei elektronischen Komponenten (102A-B) an einem elastomeren Substrat (602);
Anordnen einer elektrischen Zwischenverbindung (104), die aus einem Halbleitermaterial besteht, zwischen den zumindest zwei elektronischen Komponenten (102A-B) in einem bustrophedonen Muster, das zwischenverbundene Querstäbe (108) zwischen den zumindest zwei elektronischen Komponenten (102A-B) definiert, wobei die elektrische Zwischenverbindung (104) gleichzeitig wie die elektronischen Komponenten (102A-B) hergestellt wird;
Zwischenverbinden der zumindest zwei elektronischen Komponenten (102A-B) mittels der elektrischen Zwischenverbindung (104); und
Dehnen des elastomeren Substrats (602), sodass sich die zumindest zwei elektronischen Komponenten (102A-B) relativ voneinander entfernen.

15. Verfahren nach Anspruch 13, wobei das Halbleitermaterial ein Einkristall-Halbleitermaterial ist.

## Revendications

1. Procédé d'assemblage d'un dispositif électronique étirable, le procédé comprenant les étapes consistant à :
fixer au moins deux composants électroniques (102A-B) à un substrat élastomère (602) ;
agencer une interconnexion électrique (104) réalisée à partir d'un matériau semi-conducteur entre lesdits au moins deux composants électroniques (102A-B) selon un motif boustrophédonique qui définit des échelons (108) entre lesdits au moins deux composants électroniques (102A-B), une pluralité desdits échelons (108) étant sensiblement parallèles entre eux et ayant sensiblement la même longueur et le même déplacement entre eux, dans lequel un rapport de ladite longueur de ladite pluralité desdits échelons (108) et dudit déplacement entre ladite pluralité desdits échelons (108) est d'au moins 10:1 ;
interconnecter les au moins deux composants électroniques (102A-B) avec ladite interconnexion électrique (104) ; et
étirer le substrat élastomère (602) de telle sorte que lesdits au moins deux composants électroniques (102A-B) se séparent l'un de l'autre.

2. Procédé selon la revendication 1, dans lequel l'étirage du substrat élastomère (602) est un étirage en translation.

3. Procédé selon la revendication 1 ou 2, dans lequel la séparation entre les au moins deux composants électroniques (102A-B) augmente d'un pour cent du fait de l'étirage du substrat élastomère (602).

4. Procédé selon l'une quelconque des revendications précédentes, dans lequel le matériau semi-conducteur et le motif boustrophédonique de ladite interconnexion électrique (104) permettent à ladite interconnexion électrique (104) de maintenir des caractéristiques de performances électriques sensiblement identiques lors de la séparation entre les au moins deux composants électroniques (102A -B) augmente de 10 %.

5. Procédé selon la revendication 1, 2 ou 3, dans lequel le matériau semi-conducteur et le motif boustrophédonique de ladite interconnexion électrique (104) permettent à ladite interconnexion électrique (104) de maintenir des caractéristiques de performances électriques sensiblement identiques lorsque la séparation entre les au moins deux composants électroniques (102A-B) augmente de 100 %.

6. Procédé selon la revendication 1, 2 ou 3, dans lequel le matériau semi-conducteur et le motif boustrophédonique de ladite interconnexion électrique (104) permettent à ladite interconnexion électrique (104) de maintenir des caractéristiques de performances électriques sensiblement identiques lorsque la séparation entre les au moins deux les composants électroniques (102A-B) augmente de 1000 %.

7. Procédé selon la revendication 1, 2 ou 3, dans lequel le matériau semi-conducteur et le motif boustrophédonique de ladite interconnexion électrique (104) permettent à ladite interconnexion électrique (104) de maintenir des caractéristiques de performances électriques sensiblement identiques lorsque la séparation entre les au moins deux électroniques composants (102A-B) augmente de 10 000 %.

8. Procédé selon l'une quelconque des revendications précédentes, dans lequel les échelons (108) comprennent des barres longues (108) reliées par des barres courtes (110).

9. Procédé selon l'une quelconque des revendications précédentes, dans lequel l'étirage du substrat élastomère (602) est un étirage en rotation.

10. Procédé selon la revendication 9, dans lequel l'étirage en rotation est supérieur à 180 degrés.

11. Procédé selon l'une quelconque des revendications précédentes, dans lequel l'étirage du substrat élastomère (602) comprend l'étirage du substrat élastomère (602) dans les trois axes.

12. Procédé selon l'une quelconque des revendications précédentes, dans lequel les au moins deux composants électroniques (102A-B) sont fabriqués à partir d'un matériau semi-conducteur.

13. Procédé selon la revendication 12, dans lequel le matériau semi-conducteur de l'interconnexion électrique (104) est le même que le matériau semi-conducteur à partir duquel les au moins deux composants électroniques (102A-B) sont fabriqués.

14. Procédé d'assemblage d'un dispositif électronique étirable, le procédé comprenant les étapes consistant à :
fixer au moins deux composants électroniques (102A-B) à un substrat élastomère (602) ;
agencer une interconnexion électrique (104) réalisée à partir d'un matériau semi-conducteur entre les au moins deux composants électroniques (102A-B) selon un motif boustrophédonique qui définit des échelons interconnectés (108) entre les au moins deux composants électroniques (102A-B), dans lequel l'interconnexion électrique (104) est fabriquée en même temps que les composants électroniques (102A-B)
interconnecter les au moins deux composants électroniques (102A-B) avec l'interconnexion électrique (104) ; et
étirer le substrat élastomère (602) de telle sorte que les au moins deux composants électroniques (102A-B) se séparent l'un de l'autre.

15. Procédé selon la revendication 13, dans lequel le matériau semi-conducteur est un matériau semi-conducteur monocristallin.
